(19) Europäisches Patentamt / European Patent Office / Office européen des brevets

(11) **EP 4 740 883 A1**

(12) **EUROPEAN PATENT APPLICATION**

(43) Date of publication:
13.05.2026 Bulletin 2026/20

(51) International Patent Classification (IPC):
***A61B 18/12*** (2006.01)

(21) Application number: 25214497.7

(22) Date of filing: 10.11.2025

(52) Cooperative Patent Classification (CPC):
**A61B 18/1206; A61B 18/1233;** A61B 2018/00642;
A61B 2018/00648; A61B 2018/00666

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 11.11.2024 US 202418942992

(71) Applicant: **Biosense Webster (Israel) Ltd.**
**2066717 Yokneam (IL)**

(72) Inventors:
• **GLINER, Vadim**
**2066717 Yokneam (IL)**
• **GOVARI, Assaf**
**2066717 Yokneam (IL)**

(74) Representative: **Carpmaels & Ransford LLP
One Southampton Row
London WC1B 5HA (GB)**

(54) **TISSUE PROXIMITY INDICATOR THRESHOLD FOR CARDIAC ABLATION**

(57) In one exemplary mode, an apparatus includes a catheter including an electrode to provide an intracardiac electrogram (IEGM) signal and a signal used to sense impedance, an ablation energy generator configured to conduct ablative energy to the electrode of the catheter based on user input, a controller configured to sample the IEGM signal from the electrode before and after a given ablation, and sense impedance between the electrode and tissue, and a processor configured to compare the sensed impedance with a TPI threshold to determine a contact status of the electrode with respect to the tissue, render to a display an indication of the determined contact status, compute a change in the IEGM signal due to the given ablation, and adjust the TPI threshold for subsequent ablations based on the computed change in the IEGM signal due to the given ablation of the tissue with the electrode.

FIG. 2

**Description**

FIELD OF THE DISCLOSURE

The present disclosure relates to relates to ablation with therapeutic intracardiac catheters, and in particular, but not exclusively to, tissue proximity indication during cardiac ablation.

BACKGROUND

A wide range of medical procedures involve placing probes, such as catheters, within a patient's body. One medical procedure in which these types of probes or catheters have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium and the heart volume, and selectively ablating cardiac tissue by application of energy. Catheters are inserted into the heart chamber and optionally around the heart chamber during such procedures. In most procedures, multiple catheters are inserted into the patient. Catheters may include mapping, ablation, temperature sensing and image sensing catheters. Some catheters are dedicated for placement in specific parts of the anatomy, e.g., coronary sinus, esophagus, atrium, ventricle. The catheters have multiple electrical channels, some more than others depending on the number of sensors and electrodes included in each catheter. The number and type of catheters depends on the procedure and on the physician preferred workflow.

A typical ablation procedure involves the insertion of a catheter having a one or more electrodes at its distal end into a heart chamber. RF (radio frequency) current (or pulsed-field ablation (PFA) energy) is applied through the tip electrode(s) of the ablating catheter, and current flows through the media that surrounds it, i.e., blood and tissue, between the tip electrode(s) and an indifferent electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive.

Therefore, when placing an ablation or other catheter within the body, particularly near the endocardial tissue, it is desirable to have the ablation electrode(s) of the catheter be in direct contact with the tissue. Electrode-tissue contact may be measured based on the impedance between an electrode on a distal end of the catheter and a return electrode.

BRIEF DESCRIPTION OF THE DRAWINGS

The present disclosure will be understood from the following detailed description, taken in conjunction with the drawings in which:

Fig. 1 is a simplified illustration of a catheter-based electrophysiology mapping and ablation system constructed and operative in accordance with an example of the present disclosure;

Fig. 2 is a more detailed isometric view of an expandable distal end assembly of a catheter for use with the system of Fig. 1;

Fig. 3 is a simplified qualitative illustration showing impedance measured by an electrode in a body cavity as a function of proximity of the electrode from the cavity wall tissue for use in the system of Fig. 1; and

Figs. 4A and 4B is a flowchart including steps in a method to adjust a tissue proximity indicator threshold for use in the system of Fig. 1.

DESCRIPTION OF EXAMPLES

OVERVIEW

As previously mentioned, contact between an electrode and tissue may be measured based on impedance between the electrode and the tissue. The quality of contact between the electrode and the tissue may be expressed as a tissue proximity indicator (TPI). The TPI threshold for a particular patient may be used to determine whether the electrode is in sufficient contact with tissue in order to provide a successful ablation, for example. What is generally important from the physician standpoint to consider an electrode being in sufficient contact with tissue, is if the cardiac signal at the ablation site is sufficiently attenuated by the ablation. If the cardiac signal at the tissue is sufficiently attenuated by the ablation, after the fact, the electrode was in sufficient contact with the tissue during the ablation.

A TPI threshold may be selected and defines whether a given impedance value indicates sufficient contact for sufficiently attenuating the cardiac signal. For example, if a sensed impedance is greater than the threshold TPI, then the sensed impedance indicates that the electrode is in sufficient contact with the tissue to successfully ablate the tissue, whereas if the sensed impedance is not greater than the TPI threshold, then the sensed impedance indicates that the electrode is not in sufficient contact with the tissue. Therefore, it is important that the TPI threshold is selected carefully so that it indicates the quality of contact needed to achieve a successful ablation, e.g., a desired lesion.

In practice, the TPI threshold needed to achieve a desired lesion is generally patient dependent and also location dependent. Therefore, having a generic TPI threshold does not provide a good indicator of "contact" between the electrode and the tissue. For example, the physician may use one TPI threshold for ablating around the pulmonary vein (PV) and another TPI threshold for ablating the roof of the atrium. Another example, the physician may use one TPI threshold for ablating left superior PV (LSPV) and another for ablating the right inferior PV (RIPV). In addition, a physician may use significantly different TPI thresholds for different patients.

Examples of the present disclosure address at least some of the above challenges by providing a system in which the TPI threshold is dynamically adjusted per patient (and optionally) per cardiac location. For example, the TPI threshold may be dynamically adjusted for patient X for the PV and another TPI threshold may be dynamically adjusted for patient X for the RIPV.

Initially the TPI threshold may be estimated. Disclosed examples describe a method for estimating the initial TPI threshold based on minimum and maximum impedances. The system samples an intracardiac electrogram (IEGM) sensed by the electrode and also performs an impedance measurement to measure impedance between the electrode and the tissue. The IEGM may be sensed between the electrode and another catheter electrode or one or more body surface electrodes. The system measures impedance and compares the impedance to the TPI threshold to determine the contact status of the electrode. For example, if the impedance is greater than the TPI threshold, then the contact status is considered to be in sufficient contact, and if the measured impedance is less than the TPI threshold, the contact status is considered not to be in sufficient contact. The contact status may be rendered to a display screen for the physician to see.

The physician decides to perform ablation at the current location of the electrode based on one or more factors. The system samples the IEGM, ablation is performed, and the system samples the IEGM after ablation to determine (the amplitude or other measurement of) the reduction in the IEGM due to the ablation. IEGM reduction may be defined as reduction in magnitude of peak-peak voltage after ablation as compared to before ablation. The TPI threshold adjustment is defined as a function of a magnitude of the reduction in the IEGM or alternatively as a function of a percent reduction in voltage due to the ablation. The TPI threshold is reduced based on the reduction in the IEGM if the reduction in the IEGM is greater than a given reduction. If the reduction in the IEGM is less than or equal to the given reduction, the TPI threshold may be increased. The following formula may be used to compute the new TPI threshold:

$$\text{New TPI threshold} = \text{Old TPI threshold} - (\text{DeltaV} - \text{B})\text{A,}$$

Where DeltaV is the reduction in the voltage amplitude of the IEGM due to ablation, and A and B are threshold adjustment factors. A and B are selected to achieve a gradual change in the TPI threshold towards a target TPI threshold that will achieve a desired lesion with ablation. For example, if DeltaV is expected to be around 4 milli Volts, B may be around 0.3 or 0.4, and A may be around 5 for example. However, any suitable values of A and B may be selected, e.g., by the user. The above steps may be repeated at different ablation sites such that the TPI threshold may be adjusted after each successful ablation.

The TPI threshold may be electrode-specific such that the electrode-specific TPI threshold is dynamically adjusted due to data collected with respect to a given electrode. In some examples, the TPI threshold may be common for all (or a subset of) electrodes such that the common TPI threshold is dynamically adjusted due to data collected with respect to any given electrode (of the catheter or the subset of electrodes).

In some examples, the physician may choose to reset the TPI threshold(s) to the original threshold (before adjustment) anytime. Optionally, the system may reset the TPI threshold(s) if the distance between two subsequent ablation sites is greater than a pre-defined distance. The predefined distance may be defined by the user (e.g., physician) or the system. In this manner, the system does not require an excessive amount contact force for no reason.

SYSTEM DESCRIPTION

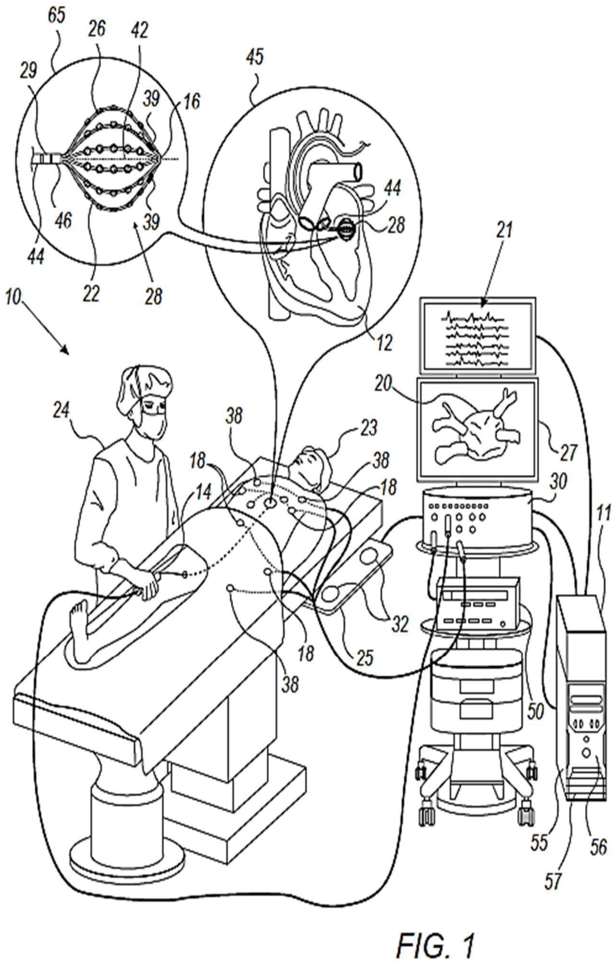

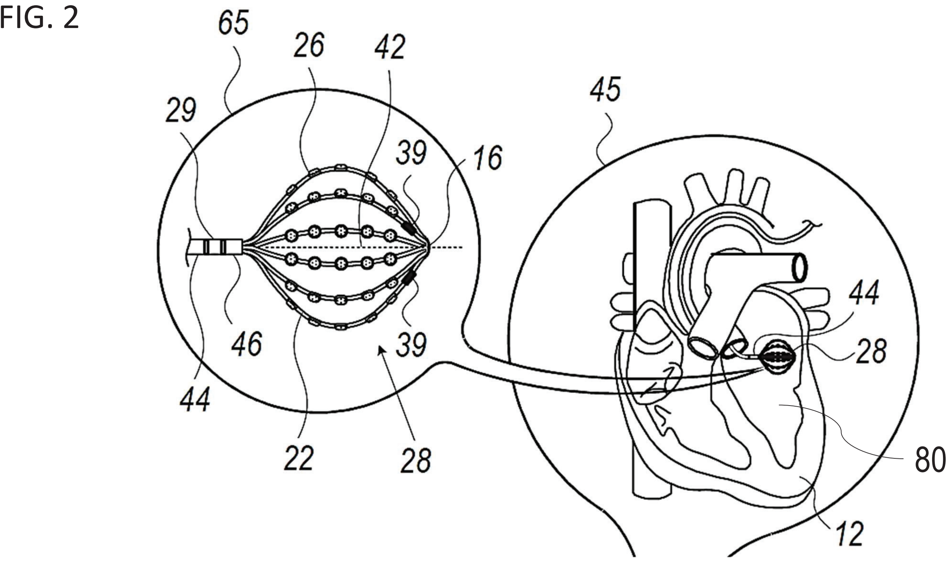

Reference is made to Fig. 1, which is a simplified illustration of a catheter-based electrophysiology mapping and ablation system, according to examples of the presently disclosed subject matter, showing an example catheter-based electrophysiology mapping and ablation system 10. Reference is also made to Fig. 2, which is a more detailed isometric view of an expandable distal end assembly 28 of a catheter 14 of Fig. 1.

System 10 includes multiple catheters, which are percutaneously inserted by a physician 24 (as seen in inset 45) through the patient's vascular system into a chamber or vascular structure of a heart 12. Typically, a delivery sheath catheter is inserted into the left or right atrium near a desired location in heart 12. Thereafter, one or more catheters may be inserted into the delivery sheath catheter so as to arrive at the desired location in heart 12. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGMs is illustrated herein. Physician 24 may place a distal end assembly 28 of catheter 14 in contact with the heart wall for sensing a target site in heart 12. For ablation, physician 24 may similarly place a distal end of an ablation catheter in contact with a target site for ablating tissue.

As seen in inset 65, catheter 14 is an exemplary catheter that includes one, and preferably multiple, electrodes 26 optionally distributed over a plurality of splines 22 at expandable distal-end assembly 28 and configured to sense IEGM signals. Catheter 14 additionally includes (i) a proximal position sensor 29 comprising two or three electromagnetic coils (EMCs)) embedded in a distal end 46 of shaft 44 near expandable distal end assembly 28, and (ii) optionally one or more distal position sensors 39 to track the position of the distal end of expandable distal end assembly 28. Optionally, and preferably, position sensors 29 and 39 are magnetic-based position sensors. The inset also shows a longitudinal axis 42 of the catheter 14 and a distal edge 16 of the distal-end assembly 28. Alternatively, catheter 14 has an elongated distal end including a plurality of ring-shaped electrodes. In this example catheter, the elongated distal end forms a loop in its neutral state that is configured, e.g., sized and shaped to be positioned around an ostium of the four pulmonary veins extending from the left atrium of a patient.

Fig. 2 provides an expanded view of the inserts 45 and 65 and shows the expandable distal-end assembly 28 in contact with tissue 80 of heart 12.

The magnetic based position sensor may be operated together with a location pad 25 including a plurality of magnetic coils 32 configured to generate magnetic fields in a predefined working volume. Real time position of distal end assembly 28 of catheter 14 may be tracked based on magnetic fields generated with location pad 25 and sensed by the magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091.

System 10 may include one or more electrode patches 38 positioned for skin contact on patient 23 to establish location reference for location pad 25 as well as impedance-based tracking of electrodes 26. For impedance sensing, electrical current is directed to electrodes 26 and sensed at electrode skin patches 38 or between a pair of electrodes 26.

A recorder 11 may record and display electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer. System 10 may include an ablation energy generator 50 that is adapted to conduct ablative energy to one or more of the electrodes at a distal tip of a catheter configured for ablating. Energy produced by ablation energy generator 50 may include, but is not limited to, radio frequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, other electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory 57, a processor unit 56 with memory or storage with appropriate operating software stored therein, and user interface capability. Workstation 55 may provide multiple functions, optionally including:

(1) modeling the endocardial anatomy in three-dimensions (3D) and rendering a 3D graphical representation of the model or anatomical map 20 for display on a display device 27;

(2) displaying on display device 27 activation sequences (or other data) compiled from recorded electrograms 21 in representative visual indicia or imagery superimposed on the rendered anatomical map 20;

(3) modeling the catheter inserted into the body in three-dimensions (3D) and rendering a 3D graphical representation of the model for display on a display device 27;

(4) displaying real-time location and orientation of multiple catheters within the heart chamber; and

(5) displaying on display device 27 sites of interest such as places where ablation energy has been applied.

One commercial product embodying elements of the system 10 is available as the CARTO™ 3 System, available from Biosense Webster, Inc., 31A Technology Drive, Irvine, CA 92618.

It is noted that tissue impedance is typically greater than that of blood, and a greater impedance value is expected to be measured at an electrode which is closer to tissue than at an electrode which is immersed in blood away from a tissue wall. In some examples tissue proximity is based on a patient-specific minimum and maximum impedance values measured in a patient.

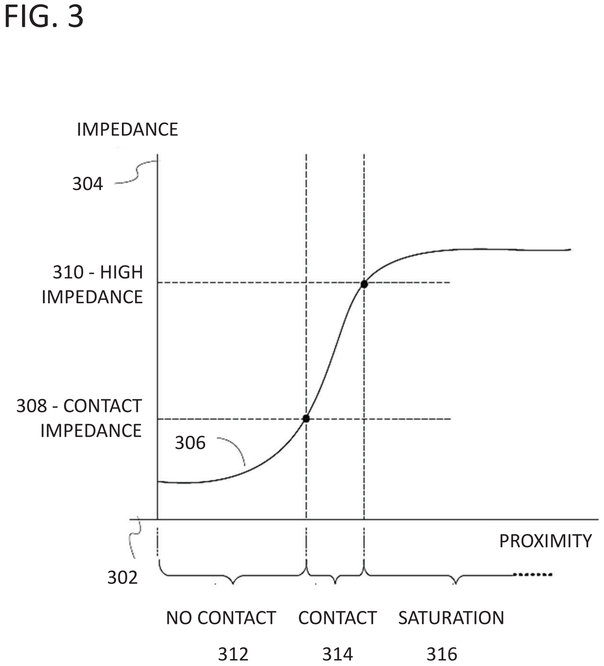

Reference is now made to Fig. 3, which is a simplified qualitative illustration showing impedance measured by an electrode in a body cavity as a function of distance from the cavity wall tissue 80 according to an aspect of the disclosure. Fig. 3 shows a graph having an X-axis 302 of qualitative proximity values (e.g., TPIs) of proximity to cavity wall tissue 80 and a Y-axis 304 of impedance values. The graph of Fig. 3 includes a line 306 which indicates impedance values for an electrode in a body cavity as a function of qualitative proximity to the cavity wall, and also a continuation of the line 306 into a region where the electrode touches the cavity wall with various amounts of pressure.

The graph of Fig. 3 shows an expected impedance behavior in a body cavity as a function of proximity. It is noted that proximity to a cavity wall increases as distance to the cavity wall decreases. The graph of Fig. 3 shows an expected shape of the impedance proximity relationship.

In some exemplary modes, in-vivo measurements may determine impedance values at end points, minimum and maximum. Such minimum and maximum values may be determined per patient, optionally by accumulating data in real-time, and determining the minimum and maximum of the real-time data.

It is noted that the minimum and maximum impedance values for each electrode are, *inter alia,* a function of the electrode's distance to a reference electrode. In some examples, the minimum and maximum impedance values per electrode are adjusted to determine normalized minimum and maximum impedance values for all the electrodes.

In some examples, data points for constructing the line 306 are optionally measured in vivo, by an electrode moving within the body cavity, measuring impedance. All or at least some of the measurements are used to produce the line 306.

In some examples, just maximum and minimum impedance values are optionally discovered, optionally per patient, by in vivo measurement. In some examples, data points for constructing the line 306 are optionally measured in vivo, by several electrodes moving within the body cavity, the electrodes measuring impedance, and optionally a position measuring system measuring locations of the electrodes.

In some examples, data points for constructing the line 306 are optionally obtained from a database which includes data measured at some historic time for a specific patient. In some examples, data points for constructing the line 306 are optionally obtained from a database which includes data measured at some historic time for a group of patients, optionally averaged.

Fig. 3 also shows two specific levels of impedance along the Y-axis 304. A first specific impedance level 308 shows an impedance of an electrode beginning to touch the cavity wall, and a distance to the cavity wall is close to zero. The line 306 to left of the contact impedance is in a "No Contact" impedance zone 312. In some examples, a value for the first specific impedance level 308 is determined as a specific percentage below the maximum of the line 306.

A second specific impedance level 310 shows an impedance of an electrode touching and pushing the cavity wall at a level of force which is considered sufficient for performing ablation using the electrode, if so desired. A corresponding "Contact" zone 314 is shown where the cavity wall is pushed somewhat from the point of contact due to more substantial force being applied to the cavity wall to produce sufficiently good contact. Beyond the "Contact" zone 314 the impedance flattens out, reaching its maximum, and this is shown by a "Saturation" zone 316, and a high impedance above a level referenced by 310.

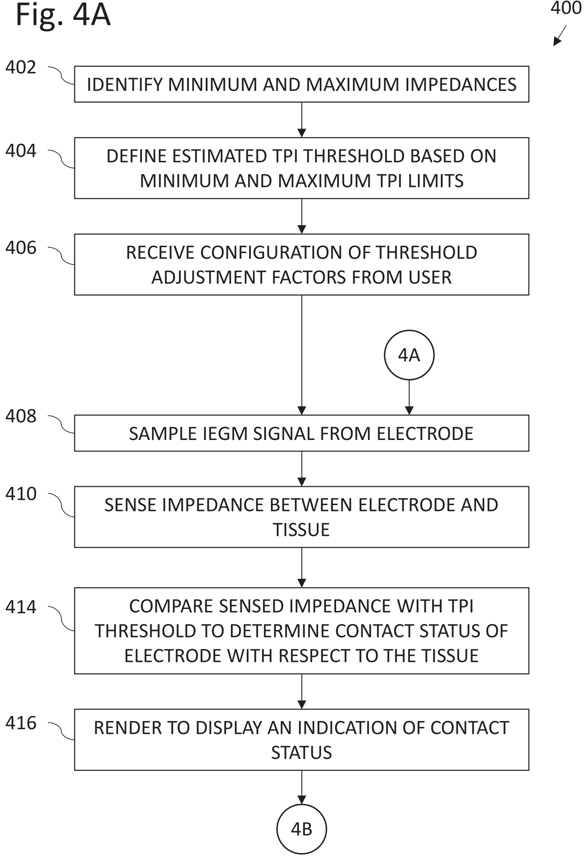

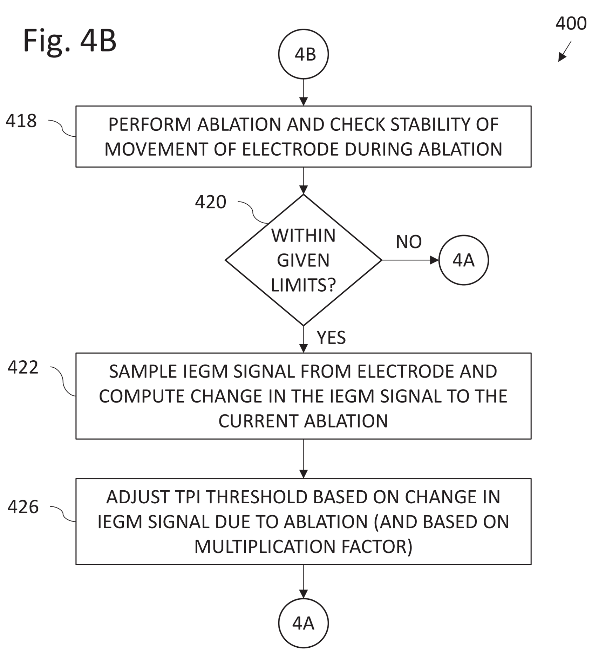

Reference is now made to Figs. 4A and 4B, which is a flowchart 400 including steps in a method to adjust a tissue proximity indicator threshold for use in the system of Fig. 1. The steps shown in Fig. 4A are first described.

In some examples, the processor unit 56 is configured to: identify minimum and maximum impedances and corresponding minimum and maximum TPI limits (block 402) for example using the method described above with reference to Fig. 3; and define an estimated TPI threshold to be between the minimum and maximum TPI limits and based on a function of the minimum and maximum TPI limits (block 404). In some examples, the estimated TPI threshold may be defined as a given distance from the minimum or maximum, or at a given ratio between the minimum and maximum TPI limits.

In some examples, the processor unit 56 is configured to receive user input to determine threshold adjustment parameters A and B (block 406), as described in more detail below.

The PIU 30 (or any suitable controller) is configured to: sample an intracardiac electrogram (IEGM) signal from a given electrode (e.g., one of the electrodes 26) (e.g., before ablation at a target tissue location) (block 408); and sense impedance between the given electrode 26 and the tissue 80 (block 410) based on impedance measured between the given electrode 26 and another catheter electrode 26 or a body surface electrode 18, for example. The catheter 14 (Fig. 1) includes the given electrode 26 which provides the IEGM signal, and a signal used to sense the impedance between the given electrode 26 and the tissue 80.

The processor unit 56 is configured to: compare the measured impedance with the (estimated or current) TPI threshold to determine a contact status of the given electrode 26 with respect to the tissue 80 (block 414). In some examples, if the measured impedance is less than or equal to the TPI threshold the contact status is not in contact, whereas if the measured impedance is greater than the TPI threshold the contact status is in contact. In other examples, if the measured impedance is less than the TPI threshold the contact status is not in contact, whereas if the measured impedance is greater than, or equal to, the TPI threshold the contact status is in contact. The contact status indicates whether the given electrode 26 is in sufficient proximity with the tissue 80 to cause a predefined reduction in the IEGM signal due to ablation. The reduction in the IEGM signal may be defined as a reduction in the IEGM signal as measured after ablation when compared to the measurement performed prior to ablation based on a peak-to-peak measurement of the IEGM signal in a window of interest of the IEGM signal.

The processor unit 56 is configured to render to display device 27 an indication of the contact status of the given electrode with respect to the tissue (block 416). The indication may include a text value (e.g., "in contact" or "not in contact") and or a color value (e.g., "red" for not in contact, and "green" for in contact).

The steps shown in Fig. 4B are now described.

The ablation energy generator 50 is configured to conduct ablative energy to the given electrode 26 of catheter 14, based on user input typically under control of the physician 24, and the processor unit 56 is configured to check the stability of movement of the given electrode 26 during ablation, for example, by monitoring the location of the given electrode 26 during the ablation (block 418).

In some examples, at a decision block 420, the processor unit 56 determines whether the stability of the given electrode 26 is within given limits (e.g., defined by the user). If the stability of the given electrode 26 is not within given limits the method returns to the step of block 408 shown on Fig. 4A where the processor unit 56 is configured to sample the IEGM signal from the given electrode 26 prior to another ablation (e.g., at a different ablation site). If the stability of the given electrode 26 is within given limits, the method continues with the step of block 422.

The PIU 30 (or any suitable controller) is configured to sample the intracardiac electrogram (IEGM) signal from the given electrode 26 (e.g., after ablation at the target tissue location) (block 422) and the processing unit is configured to compute a change in (the peak-to-peak voltage of) the IEGM signal due to the current ablation.

The processor unit 56 is configured to adjust the TPI threshold for subsequent ablations based on the computed change in the IEGM signal due to the current ablation of the tissue 80 with the given electrode 26 (block 426). When the stability of the given electrode is checked in the step of block 418, the step of block 426 is therefore dependent upon the stability of movement of the given electrode 26 being within a given limit or limits. The TPI threshold is adjusted based on the reduction in the IEGM. The TPI threshold is reduced based on the reduction in the IEGM if the observed reduction in the IEGM is greater than a given reduction. If the observed reduction in the IEGM is less than or equal to the given reduction, the TPI threshold may be increased. The adjustments in the TPI threshold allow the TPI threshold to be gradually adjusted until the TPI threshold reaches or approaches the target TPI threshold. The following formula may be used to compute the new TPI threshold:

$$\text{New TPI threshold} = \text{Old TPI threshold} - (\text{DeltaV} - B)A,$$

Where DeltaV is the reduction in the voltage amplitude of the IEGM due to ablation, and A and B are threshold adjustment factors. A and B are selected to achieve a gradual change in the TPI threshold towards the target "real" TPI threshold. For example, if DeltaV is expected to be around 4 milli Volts, B may be around 0.3 or 0.4, and A may be around 5 for example. However, any suitable values of A and B may be selected, e.g., by the user.

In some exemplary modes, the processor unit 56 is configured to adjust the TPI threshold per electrode 26 of the catheter 14. In other words, the system 10 manages multiple TPI thresholds which are electrode-specific and adjusted based on data of respective electrodes 26. In other exemplary modes, the processor unit 56 is configured to adjust the TPI threshold such that the same TPI threshold is maintained for multiple electrodes 26 of the catheter 14.

The processor unit 56 is configured to iteratively adjust TPI thresholds based on respective previous TPI thresholds and respective reductions in voltage of the IEGM signal due to ablation at respective ablation sites. In other words, the steps of blocks 408 to 426 are repeated at different ablation sites and the TPI threshold may be adjusted at the step of block 426 after each ablation in order to converge toward an optimal TPI threshold that best indicates the proximity needed to achieve the desired reduction in peak-to-peak IEGM voltage due to ablation (or a desired lesion).

In practice, some or all of these functions of processor unit 56 may be combined in a single physical component or, alternatively, implemented using multiple physical components. These physical components may comprise hardwired or programmable devices, or a combination of the two. In some examples, at least some of the functions of the processor unit 56 may be carried out by a programmable processor under the control of suitable software. This software may be downloaded to a device in electronic form, over a network, for example. Alternatively, or additionally, the software may be stored in tangible, non-transitory computer-readable storage media, such as optical, magnetic, or electronic memory.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values $\pm20\%$ of the recited value, e.g., "about 90%" may refer to the range of values from 72% to 108%.

EXAMPLES

Example 1: An apparatus, comprising: a catheter including an electrode to provide an intracardiac electrogram (IEGM) signal and a signal used to sense impedance; an ablation energy generator configured to conduct ablative energy to the electrode of the catheter based on user input; a controller configured to: sample the IEGM signal from the electrode before and after a given ablation; and sense impedance between the electrode and tissue; and a processor configured to: compare the sensed impedance with a TPI threshold to determine a contact status of the electrode with respect to the tissue; render to a display an indication of the determined contact status; compute a change in the IEGM signal due to the given ablation; and adjust the TPI threshold for subsequent ablations based on the computed change in the IEGM signal due to the given ablation of the tissue with the electrode.

Example 2: The apparatus according to example 1, wherein the processor is configured to iteratively adjust TPI thresholds based on respective previous TPI thresholds and respective reductions in voltage of the IEGM signal due to ablation at respective ablation sites.

Example 3: The apparatus according to example 1 or 2, wherein the processor is configured to: check a stability of movement of the electrode during ablation; and adjust the TPI threshold based on the stability of movement of the electrode being within a given limit.

Example 4: The apparatus according to any of examples 1-3, wherein the processor is configured to decrease the TPI threshold based on observing at least a given reduction in the IEGM signal due to ablation of the tissue with the electrode.

Example 5: The apparatus according to any of examples 1-3, wherein the processor is configured to increase the TPI threshold based on observing less that a given reduction in the IEGM signal due to ablation of the tissue with the electrode.

Example 6: The apparatus according to any of examples 1-5, wherein the processor is configured to receive user input to determine threshold adjustment factors.

Example 7: The apparatus according to any of examples 1-6, wherein the processor is configured to: identify minimum and maximum impedances and corresponding minimum and maximum TPI limits; and define an estimated TPI threshold to be between the minimum and maximum TPI limits and based on a function of the minimum and maximum TPI limits.

Example 8: The apparatus according to any of examples 1-7, wherein the processor is configured to adjust the TPI threshold per electrode of the catheter.

Example 9: The apparatus according to any of examples 1-7, wherein the processor is configured to adjust the TPI threshold such that a same TPI threshold is maintained for multiple electrodes of the catheter.

Example 10: The apparatus according to any of examples 1-9, wherein the processor is configured to compute a new TPI threshold based on a previous TPI threshold less a first factor multiplied by: a reduction in a voltage of the IEGM signal less a second factor.

Example 11: A method, comprising: providing by an electrode of a catheter an intracardiac electrogram (IEGM) signal and a signal used to sense impedance; conducting ablative energy to the electrode of the catheter based on user input; sampling the IEGM signal from the electrode before and after a given ablation; sensing impedance between the electrode and tissue; comparing the sensed impedance with a TPI threshold to determine a contact status of the electrode with respect to the tissue; rendering to a display an indication of the determined contact status; computing a change in the IEGM signal due to the given ablation; and adjusting the TPI threshold for subsequent ablations based on the computed change in the IEGM signal due to the given ablation of the tissue with the electrode.

Example 12: The method according to example 11, further comprising iteratively adjusting TPI thresholds based on respective previous TPI thresholds and respective reductions in voltage of the IEGM signal due to ablation at respective ablation sites.

Example 13: The method according to example 11 or 12, further comprising checking a stability of movement of the electrode during ablation, and wherein the adjusting includes adjusting the TPI threshold based on the stability of movement of the electrode being within a given limit.

Example 14: The method according to any of examples 11-13, further comprising decreasing the TPI threshold based on observing at least a given reduction in the IEGM signal due to ablation of the tissue with the electrode.

Example 15: The method according to any of examples 11-13, further comprising increase the TPI threshold based on observing less that a given reduction in the IEGM signal due to ablation of the tissue with the electrode.

Example 16: The method according to any of examples 11-15, further comprising receiving user input to determine threshold adjustment factors.

Example 17: The method according to any of examples 11-16, further comprising: identifying minimum and maximum impedances and corresponding minimum and maximum TPI limits; and defining an estimated TPI threshold to be between the minimum and maximum TPI limits and based on a function of the minimum and maximum TPI limits.

Example 18: The method according to any of examples 11-17, wherein the adjusting includes adjusting the TPI threshold per electrode of the catheter.

Example 19: The method according to any of examples 11-17, wherein the adjusting includes adjusting the TPI threshold such that a same TPI threshold is maintained for multiple electrodes of the catheter.

Example 20: The method according to any of examples 11-19, further comprising computing a new TPI threshold based on a previous TPI threshold less a first factor multiplied by: a reduction in a voltage of the IEGM signal less a second factor.

Various features of the disclosure which are, for clarity, described in the contexts of separate examples may also be provided in combination in a single example. Conversely, various features of the disclosure which are, for brevity, described in the context of a single example may also be provided separately or in any suitable sub-combination.

The examples described above are cited by way of example, and the present disclosure is not limited by what has been particularly shown and described hereinabove. Rather the scope of the disclosure includes both combinations and subcombinations of the various features described hereinabove, as well as variations and modifications thereof which would occur to persons skilled in the art upon reading the foregoing description and which are not disclosed in the prior art.

**Claims**

1. An apparatus, comprising:

   a catheter (14) including an electrode (26) to provide an intracardiac electrogram (IEGM) signal and a signal used to sense impedance;
   an ablation energy generator (50) configured to conduct ablative energy to the electrode (26) of the catheter (14) based on user input;
   a controller (30) configured to:

      sample the IEGM signal from the electrode (26) before and after a given ablation; and
      sense impedance between the electrode (26) and tissue (80);
      and

   a processor (56) configured to:

      compare the sensed impedance with a tissue proximity indicator (TPI) threshold to determine a contact status of the electrode (26) with respect to the tissue (80);
      render to a display (27) an indication of the determined contact status;
      compute a change in the IEGM signal due to the given ablation; and
      adjust the TPI threshold for subsequent ablations based on the computed change in the IEGM signal due to the given ablation of the tissue (80) with the electrode (26).

2. The apparatus according to claim 1, wherein the processor (56) is configured to iteratively adjust TPI thresholds based on respective previous TPI thresholds and respective reductions in voltage of the IEGM signal due to ablation at respective ablation sites.

3. The apparatus according to claim 1 or 2, wherein the processor (56) is configured to:

   check a stability of movement of the electrode (26) during ablation; and
   adjust the TPI threshold based on the stability of movement of the electrode (26) being within a given limit.

4. The apparatus according to any one of claims 1 to 3, wherein the processor (56) is configured to decrease the TPI threshold based on observing at least a given reduction in the IEGM signal due to ablation of the tissue (80) with the electrode (26).

5. The apparatus according to any one of claims 1 to 4, wherein the processor (56) is configured to increase the TPI threshold based on observing less than a given reduction in the IEGM signal due to ablation of the tissue (80) with the electrode (26).

6. The apparatus according to any one of claims 1 to 5, wherein the processor (56) is configured to receive user input to determine threshold adjustment factors.

7. The apparatus according to any one of claims 1 to 6, wherein the processor (56) is configured to:

identify minimum and maximum impedances and corresponding minimum and maximum TPI limits; and define an estimated TPI threshold to be between the minimum and maximum TPI limits and based on a function of the minimum and maximum TPI limits.

8. The apparatus according to any one of claims 1 to 7, wherein the processor (56) is configured to adjust the TPI threshold per electrode (26) of the catheter (14).

9. The apparatus according to any one of claims 1 to 7, wherein the processor (56) is configured to adjust the TPI threshold such that a same TPI threshold is maintained for multiple electrodes (26) of the catheter (14).

10. The apparatus according to any one of claims 1 to 9, wherein the processor (56) is configured to compute a new TPI threshold based on a previous TPI threshold less a first factor multiplied by: a reduction in a voltage of the IEGM signal less a second factor.

FIG. 1

FIG. 2

FIG. 3

IMPEDANCE

304

310 - HIGH IMPEDANCE

308 - CONTACT IMPEDANCE

306

302

PROXIMITY

NO CONTACT

312

CONTACT

314

SATURATION

316

# Fig. 4A

400

402 — IDENTIFY MINIMUM AND MAXIMUM IMPEDANCES

404 — DEFINE ESTIMATED TPI THRESHOLD BASED ON MINIMUM AND MAXIMUM TPI LIMITS

406 — RECEIVE CONFIGURATION OF THRESHOLD ADJUSTMENT FACTORS FROM USER

( 4A )

408 — SAMPLE IEGM SIGNAL FROM ELECTRODE

410 — SENSE IMPEDANCE BETWEEN ELECTRODE AND TISSUE

414 — COMPARE SENSED IMPEDANCE WITH TPI THRESHOLD TO DETERMINE CONTACT STATUS OF ELECTRODE WITH RESPECT TO THE TISSUE

416 — RENDER TO DISPLAY AN INDICATION OF CONTACT STATUS

( 4B )

Fig. 4B

400

4B

418 — PERFORM ABLATION AND CHECK STABILITY OF MOVEMENT OF ELECTRODE DURING ABLATION

420 — WITHIN GIVEN LIMITS?

NO → 4A

YES

422 — SAMPLE IEGM SIGNAL FROM ELECTRODE AND COMPUTE CHANGE IN THE IEGM SIGNAL TO THE CURRENT ABLATION

426 — ADJUST TPI THRESHOLD BASED ON CHANGE IN IEGM SIGNAL DUE TO ABLATION (AND BASED ON MULTIPLICATION FACTOR)

4A

Europäisches
Patentamt

European
Patent Office

Office européen
des brevets

# EUROPEAN SEARCH REPORT

**Application Number**

EP 25 21 4497

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2019/126260 A1 (ST JUDE MEDICAL CARDIOLOGY DIV INC [US]) 27 June 2019 (2019-06-27) * the whole document * ----- | 1-10 | INV. A61B18/12 |
| A | EP 4 374 810 A1 (BIOSENSE WEBSTER ISRAEL LTD [IL]) 29 May 2024 (2024-05-29) * the whole document * ----- | 1-10 | |
| A | US 2009/306643 A1 (PAPPONE CARLO [IT] ET AL) 10 December 2009 (2009-12-10) * the whole document * ----- | 1-10 | |

| | | | TECHNICAL FIELDS SEARCHED (IPC) |
|---|---|---|---|
| | | | A61B |

The present search report has been drawn up for all claims

| Place of search | Date of completion of the search | Examiner |
|---|---|---|
| Munich | 2 March 2026 | Wetzig, Thomas |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 21 4497

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-2026

| Patent document cited in search report | Publication date | Patent family member(s) | Publication date |
|---|---|---|---|
| WO 2019126260 A1 | 27-06-2019 | CN 111479497 A | 31-07-2020 |
| | | EP 3668381 A1 | 24-06-2020 |
| | | JP 7106644 B2 | 26-07-2022 |
| | | JP 2021528108 A | 21-10-2021 |
| | | US 2019183378 A1 | 20-06-2019 |
| | | US 2019274581 A1 | 12-09-2019 |
| | | US 2023225678 A1 | 20-07-2023 |
| | | WO 2019126260 A1 | 27-06-2019 |
| EP 4374810 A1 | 29-05-2024 | CN 118021444 A | 14-05-2024 |
| | | EP 4374810 A1 | 29-05-2024 |
| | | IL 308352 A | 01-06-2024 |
| | | JP 2024071364 A | 24-05-2024 |
| | | US 2024156520 A1 | 16-05-2024 |
| US 2009306643 A1 | 10-12-2009 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5391199 A
- US 5443489 A
- US 5558091 A
- US 6172499 B
- US 6239724 B
- US 6332089 B
- US 6484118 B
- US 6618612 B
- US 6690963 B
- US 6788967 B
- US 6892091 B